# EUROPEAN PATENT APPLICATION

(11) **EP 2 647 401 A1**
(43) Date of publication of application: **09.10.2013**
(21) Application number: 12163230.1
(22) Date of filing: 04.04.2012
(51) Int. Cl.: A61M 16/10, B01D 46/24, B01D 46/00, B01D 46/52, B01D 46/12

(54) **A filter of absorbing, retaining and releasing heat and/or moisture of exhaled and inhaled gas and method of forming such a filter**

(71) Applicant: Covidien AG, 8212 Neuhausen am Rheinfall (CH)
(72) Inventor: Tralli, Stefano, 46022 Felonica (MN) (IT)
(74) Representative: Olsson, Carl H.S.

(57) **Abstract**

The invention relates to a filter (10, 80, 90) capable of absorbing, retaining and releasing heat and/or moisture of exhaled and inhaled gas, preferably for use in a medical heat and moisture exchanger (HME). The filter (10, 80, 90) has at least one sheet (20, 70) of filter material comprising a plurality of elevations (40, 42; 72, 74). Further, the invention relates to a method of forming such a filter (10, 80, 90) by pressing a pattern on the at least one sheet (20, 70) to form the elevations (40, 42; 72, 74).

## Description

The invention relates to a filter capable of absorbing, retaining and releasing heat and/or moisture of exhaled and inhaled gas, preferably for use in a medical heat and moisture exchanger (HME). The filter comprises at least one sheet of filter material. Further, the invention relates to a method of forming such a filter.

The filter device according to the present invention can be used in a breathing system for use in a patient, for example in intensive care or intubated patients undergoing artificial respiration.

Usually, a respiratory gas humidification system comprises a heat and moisture exchanger (HME) for conditioning the respiratory gas for the patient by absorbing, retaining and releasing heat and/or moisture of exhaled and inhaled gas. Respiratory gas humidification is a method of artificially conditioning respiratory gas for the patient during therapy and involves humidification, warming and, occasionally, filtration of the gas being delivered. If these three measures are not performed, pulmonary infections and lung tissue damage may occur.

WO 2006/129124 A2 discloses a method of manufacturing a filter comprising the steps of introducing the filter into a first housing component, applying a bead of adhesive to the first housing component or a part of the filter and engaging the first and the second housing components together such that pressure is applied to the bead of adhesive. The housing components are adapted such that application of pressure to the bead of adhesive causes adhesive to be displaced into contact with the filter and/or with at least one of the housing components, the adhesive thereby forming a seal between the filter and at least one of the housing components. Further, this document discloses a filter medium having a sheet of filter material that is pleated and bonded together.

EP 2 332 630 A1 discloses a gas filter for a breathing system, the filter comprising a sheet of material arranged in a plurality of pleats extending transversally across the filter. Each pleat comprises two side walls and an end face, the side walls of the filter being arranged such that they extend parallel to each other over substantially the whole height of the pleats. Preferably, the end face of each pleat is square to the side walls.

It is an object of the present invention to provide a filter as mentioned above, preferably for use in a medical heat and moisture exchanger, having both a simple configuration and a good performance as regards the conditioning of the gas flowing through the filter. In other words the filter should be of an easy construction allowing repeatability in the production of the filters with continuous characteristics, in particular as to the conditioning performance.

The above object is achieved by a filter according to claim 1. According to the present invention, the filter capable of absorbing, retaining and releasing heat and/or moisture of exhaled and inhaled gas, preferably for use in a medical heat and moisture exchanger (HME) comprises at least one sheet of filter material, wherein the at least one sheet of filter material comprises a plurality of elevations.

As to the terms used in the present invention, the filter can also be designated as filter medium, filter membrane, gas filter for a breathing system, breathing filter, respiratory filter, respiratory gas filter or respiratory gas humidifier.

The elevations may also be designated as projections or protrusions. Preferably, the elevations extend in a direction away from the extension plane of the sheet of filter material. In this regard it is to be mentioned that the elevations can have substantially identical or different heights. Preferably, the elevations may extend along a line or curve substantially perpendicular or inclined relative to the extension direction of the sheet. Preferably, this extension direction is a longitudinal direction of the sheet.

The elevations are configured and/or shaped such that, the filter provides a good gas conditioning performance over its whole cross-section. Generally speaking, the elevations function as spacers such that opposing surface regions of the sheet of filter material are spaced apart from each other to define a flow passageway for the gas to be filtered. The opposing surface regions can be provided by a first (e.g. upper) or a second (e.g. lower) side of the sheet or by both the first and second side of a single sheet or by a side of a first sheet and a side of a second sheet.

Basically, the at least one sheet of filter material may have sections with elevations and sections without elevations. These sections can follow one after another or can be positioned adjacent to each other.

Generally speaking, the elevations can be shaped substantially identical or different to each other. Further, elevations within a first section of the sheet can be of a first type of shape and/or size and elevations within a second section of the sheet can be of a second type of shape and/or size. In particular, the distance between adjacent or all extensions and/or the height of adjacent or all extensions can be identical.

Preferably, the sheet is a strip or band extending along a longitudinal direction. Preferably, this strip or band has a rectangular shape. The at least one sheet can be made of a compressible material, preferably paper (cellulose) or foam. In particular, the paper is a hygroscopic paper. The sheet can comprise only one or a plurality of layers or membranes of equal or different material. Further, the sheet or at least one layer thereof can be coated.

Preferred embodiments of the filter according to the present invention are described in claims 2 to 15.

According to a preferred embodiment, the at least one sheet comprises a first side and a second side, wherein the elevations extend away from the first side and/or second side. For example, it is possible that the elevations extend away only from the first side or only from the second side. Further, it is possible that the elevations extend alternately away from the first side and the second side. Further, the elevations can be divided in groups of elevations, e.g. a first group of elevations extend away from the first side and a second group of elevations extend away from the second side.

In a preferred embodiment, the elevations are formed integrally with the sheet. Preferably, the elevations are made of the same material as the sheet. In other words, the elevations can be provided by forming of the sheet.

In a further preferred embodiment, the elevations extend in form of at least one line or curve in an extension direction on the sheet. Preferably, the line or curve extends transverse or inclined in relation to the longitudinal direction of the sheet. For example, the line can extend in a linear manner from one side edge to the other side edge of the sheet and can be orientated transverse, e.g. diagonal, in relation to the longitudinal direction of the sheet. Further, a first group of elevations may extend along a first extension direction and a second group of elevations may extend along a second extension direction.

Preferably, the at least one line or curve comprises at least one section extending in an angle relative to a longitudinal direction of the sheet. For example, the line can be divided in several linear sections connected to each other, wherein one or more section(s) can be orientated transverse, e.g. diagonal, in relation to the longitudinal direction of the sheet. In its entirety the complete line extends in a linear manner from one side edge to the other side edge of the sheet. Preferably, the line or curve comprises two sections with different directions of extension. For example, the two sections can be orientated to form V-shaped elevations. In case of a number of sections, the different sections can extend in different angles.

According to a further preferred embodiment, the elevations are formed by corrugations. Preferably, these corrugations extend along the at least one line or curve. In other words, the at least one sheet of filter material can be corrugated or waved to provide the elevations.

According to a preferred embodiment, the filter comprises at least two sheets of filter material arranged one upon the other. Preferably, the different sheets are arranged one upon the other such that the adjacent sheets contact each other only in the areas of at least some of the opposing elevations. In this way, a sufficient cross-section for the gas passageway can be provided between two adjacent sheets of filter material. The sheets of material may be connected to each other, e.g. at predetermined locations.

In a further preferred embodiment, the elevations of the adjacent sheets are directed in different directions relative to each other. When using two sheets of filter material, arranged one upon the other, the two sheets can be positioned such that the elevations of a first sheet are directed in a first direction and the elevations of the second sheet are directed in a second direction, wherein the two directions extend in opposite directions.

In a further preferred embodiment, the at least one sheet has a plurality of pleats. Preferably, the filter consists of one sheet of filter material having a plurality of pleats.

According to a preferred embodiment, the at least one sheet has a longitudinal direction, wherein the pleats extend transverse to the longitudinal direction. Preferably, the pleats extend substantially perpendicular to the longitudinal direction of the sheet. Preferably, the pleats have acute angles and/or are substantially Z-, V-, or U-shaped. The acute angle can be between 0° and 90°, in particular between about 0° and 30°.

In a preferred embodiment, the filter is realised with only one strip of cellulose material (for example paper). In a first step this strip is corrugated to form the elevations. Preferably, the elevations are V-shaped. Second, the strip is folded (pleated) in a zigzag shape to obtain a square or rectangular shape. Preferably, the strip is folded such that the pleats are V- or Z-shaped. In this way at least some or nearly all elevations of opposing sections of the sheet contact each other and, thus, provide a gas passageway having a suitable cross-section. In this embodiment, the elevations can be formed over the whole length of the strip, which is easy to manufacture. Due to the pleats and the V-shaped elevations, the elevations of a sheet section extend in the opposite direction in relation to the elevations of an opposing section of the sheet. Hence, it is possible to manufacture a series of filters having the same properties as regards the conditioning performance and flow cross-section.

In a second preferred embodiment of the invention, at least two sheets of filter material are used, wherein the sheets have no pleats. First, a strip of sheet material is corrugated to have the V-shaped elevations as mentioned above. In addition, a second strip is corrugated in the same way. Subsequently, the two strips are arranged one upon the other such that the V-shaped elevations of the first strip extend in the longitudinal direction of the strip and the V-shaped elevations of the second strip extend in the opposite direction of the strip. In a further step, the two sheets arranged one upon the other are rolled up to obtain a round- or oval-shaped filter device. In this way at least some or nearly all elevations of opposing sections of the sheet contact each other and, thus, provide a gas passageway having a suitable cross-section.

The above object is also achieved by a medical heat and moisture exchanger (HME) comprising a casing with two connection ports and at least one filter according to one of the claims 1 to 15. The at least one filter is arranged within the casing such that exhaled or inhaled gas can flow through the filter to absorb, retain and release heat and/or moisture of the gas. Preferably, the casing comprising a first (upper) lid and a second (lower) lid, each having one connection port or connection tube.

Further, the above object is also achieved by a method of forming a filter according to any one of the claims 12 to 15, wherein the method comprises pressing a pattern on the at least one sheet to form the elevations. The step of pressing a pattern can be carried out by using a plate or using at least one roller.

Preferably, the step of pressing is performed by embossing the pattern on the sheet, in particular by passing or conveying the sheet between two rollers.

In a preferred embodiment, the method comprises folding the sheet against a blade or rolling up the at least one sheet. Preferably, the sheet is rolled up such that at least some of the elevations contact each other. As a result, opposing regions of the sheet are spaced apart from each other.

In a further preferred embodiment, at least two sheets of the filter material are laid one upon the other to build a sandwich assembly such that the elevations of the adjacent sheets are directed in different directions relative to each other. Subsequently, the sandwich assembly is rolled up.

The invention will be explained in more detail in the following with reference to the drawings, wherein:
- Fig. 1: is a perspective view of a filter according to the present invention;
- Fig. 2: is a partial enlarged plan view of the filter according to Fig. 1;
- Fig. 3: is a partial cross-sectional view of the filter along line III-III in Fig. 2.
- Fig. 4: is a perspective view showing the filter according to Fig. 1 in a pleated form;
- Fig. 5: is a side view of the pleated filter according to Fig. 2;
- Fig. 6: is an exploded view of a medical heat and moisture exchanger (HME) comprising a filter according to Fig. 1 to 5;
- Fig. 7 to 9: show an alternative embodiment of a medical heat and moisture exchanger (HME) comprising a filter according to Fig. 1 to 5;
- Fig. 10 to 12: show partial plan views similar to Fig. 2 showing alternative directions of extension of the elevations;
- Fig. 13: is a perspective view showing two sheets of a filter material according to Fig. 1 to 3 to be arranged one upon the other;
- Fig. 14: is partial enlarged cross-sectional view of the two sheets according to Fig. 13 arranged one upon;
- Fig. 15: is a front view of a filter comprising the two sheets of filter material according to Fig. 13 rolled up to have a round shape, and
- Fig. 16: is a front view of a filter comprising two sheets of filter material according to Fig. 13 rolled up and crushed to have an oval shape.

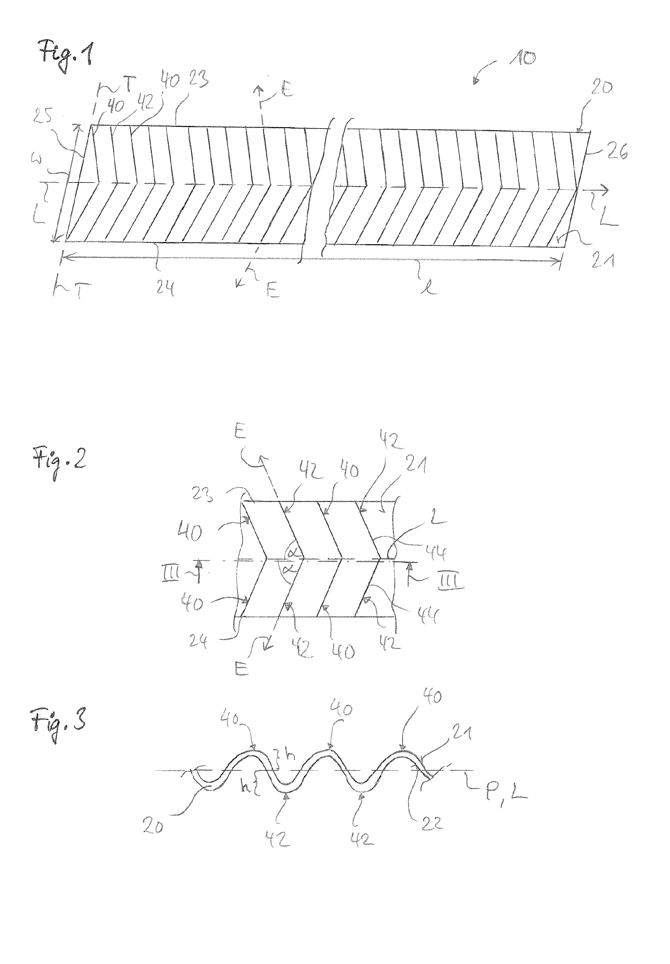

Fig. 1 to 5 show a preferred embodiment of a filter 10 capable of absorbing, retaining and releasing heat and/or moisture of exhaled and inhaled gas, preferably for use in a medical heat and moisture exchanger (HME). Fig. 2 shows a partial plan view of the filter 10 and Fig. 3 is a cross-section of the filter 10 along line III-III in Fig. 2.

The filter 10 comprises one sheet 20 of a filter material. This sheet 20 is made of cellulose-based paper. The sheet 20 has a first side 21 and a second side 22, wherein in the present embodiment the first side 21 is the upper side and the second side 22 is the lower side. In the present embodiment, the sheet 20 is a strip of material extending along a longitudinal direction L and has a rectangular shape with a length I and a width w. This strip has a first side edge 23, a second side edge 24, a first end 25 and a second end 26. The sheet extends along an horizontal extension plane P, as can be taken from Figs. 1 and 3.

As can be taken from Fig. 1 to 4, the sheet 20 comprises a plurality of elevations 40 and 42 distributed along the longitudinal direction L or length I of the sheet 20. Each elevation 40, 42 extends away from the extension plane P and has a height h. Further, each elevation 40 and 42 extends in form of a line in an extension direction E on/across the sheet 20. Basically, as can be taken from the perspective view according to Fig. 1 and the plan view according to Fig. 2, the elevations are V-shaped. A first group of elevations 40 extend substantially vertical away from the first side 21 (or extension plane P) and the second group of elevations 42 extend substantially vertical away from the second side 22 (or extension plane P). Thus, in the longitudinal direction L the elevations 40, 42 extend alternately away from the first side 21 and the second side 22. As shown in Fig. 1 and 2, each extension line of the elevations 40, 42 comprises two sections 44. Each section 44 extends in an angle α relative to the longitudinal direction L.

The elevations 40, 42 are formed integrally with the sheet 20 and are made of the same material as the sheet 20. Preferably, the elevations 40, 42 are formed by pressing a pattern on the sheet 20, in particular by embossing the pattern on the sheet by conveying the sheet 20 between two rollers. As can be taken from Fig. 3, the elevations 40, 42 are preferably formed by corrugations, that means the sheet 20 is corrugated.

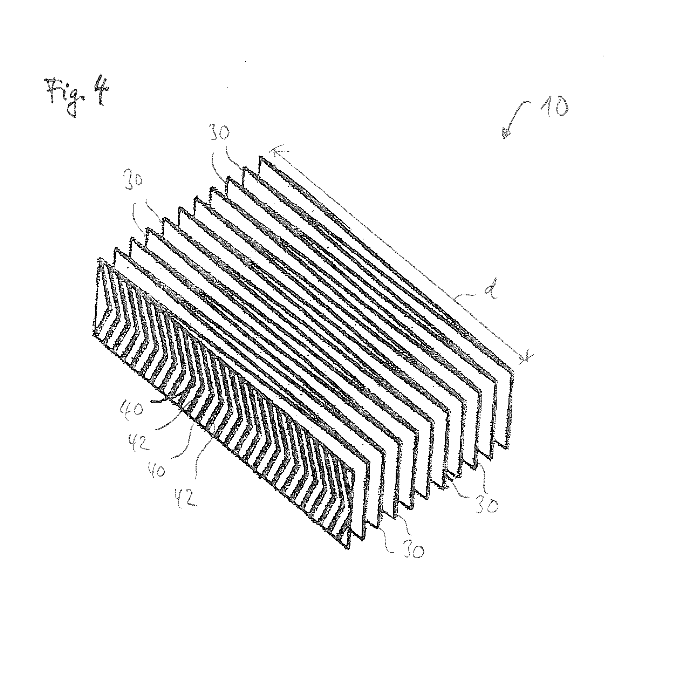

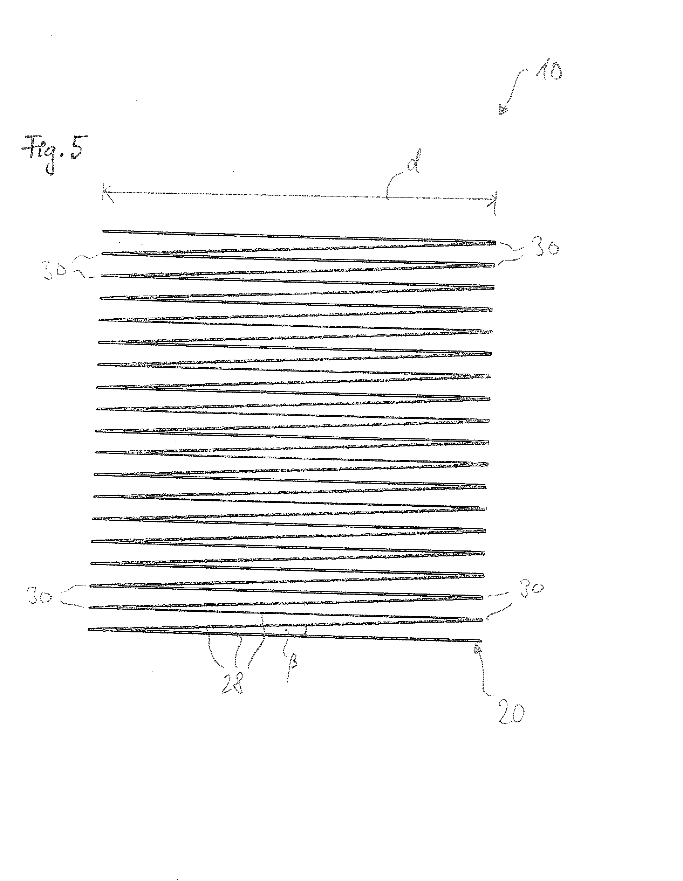

In order to provide a medical heat and moisture exchanger, the sheet 20 is folded (pleated) in a zigzag shape to obtain a square or rectangular shaped filter device as shown in Fig. 4 and 5. Preferably, the sheet 20 is folded such that the pleats 30 are Z-shaped. After the step of folding the sheet 20 has a plurality of pleats 30. The pleats 30 are distributed along the longitudinal direction L and extend in a transverse direction T (cf. Fig. 1) substantially perpendicular relative to the longitudinal direction L. The pleats 30 are spaced apart from each other by a distance d. The pleats 30 divide the sheet in a plurality of sheet sections 28 (cf. Fig. 5). As can be taken from Fig. 4 to 6, the pleats 30 have acute angles β, for example in a range between about 0 and 30°, and are substantially Z-shaped. Alternatively, the pleats 30 can be V- or U- shaped.

Due to the V-shaped elevations 40, 42 and the Z-shaped pleats 30 the elevations 40, 42 of one sheet section 28 extend in the opposite direction in relation to the elevations 40, 42 of an opposing sheet section 28. Further, at least some or nearly all elevations 40, 42 of opposing sheet sections 28 contact each other and, thus, provide a gas passageway having a suitable cross-section. In other words, the elevations 40, 42 function as spacers such that the surface regions between the elevations 40, 42 are spaced apart from each other to define a flow passageway for the gas to be filtered. Hence, it is possible to manufacture a series of filters 10 having the same properties as regards the conditioning performance and flow cross-section.

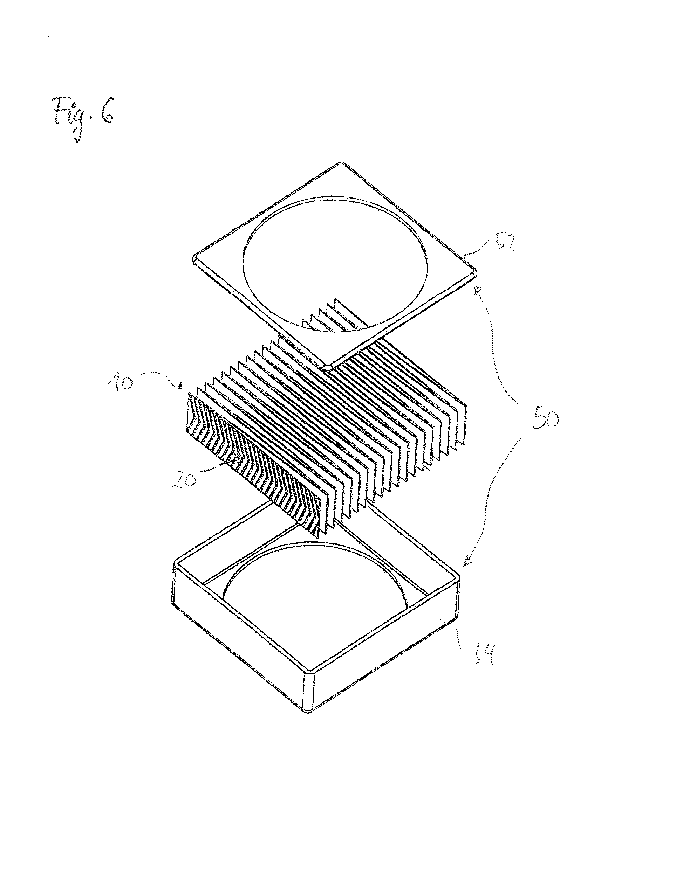

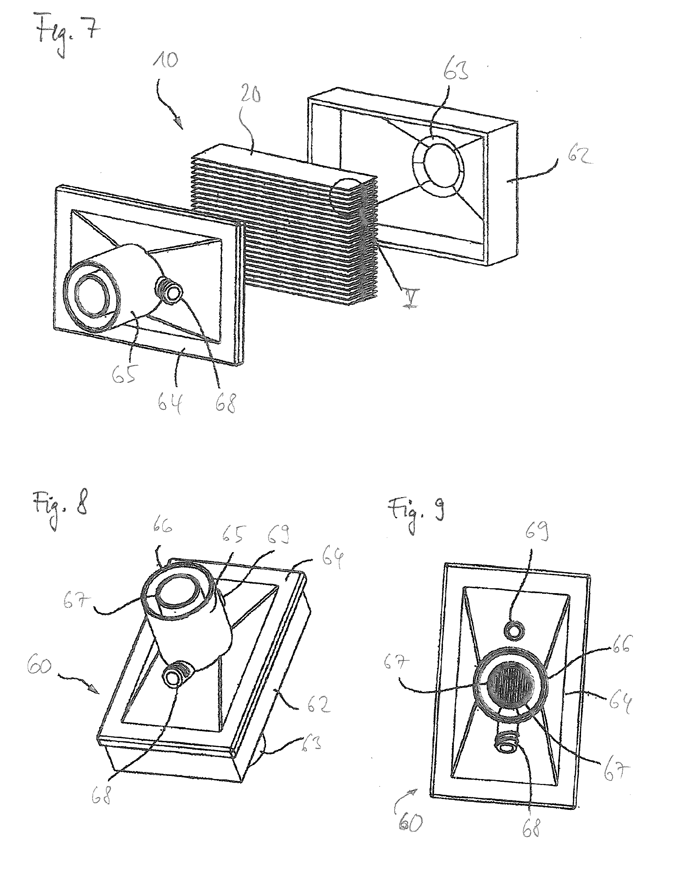

Fig. 6 shows an embodiment of a medical heat and moisture exchanger (HME) comprising a casing 50 with a first lid 52 and a second lid 54. The lids 52, 54 are configured to be coupled together in order to enclose the pleated filter 10 of Fig. 4, 5. Fig. 7 to 9 show an alternative embodiment of a medical heat and moisture exchanger (HME). This HME comprises a casing 60 with a first lid 62 and a second lid 64. Lid 62 comprises a connector tube 63 and lid 64 comprises a connector tube 65. Each connector tube 63, 65 has an outer wall 66 and an inner wall 67 to make them compatible to be connected to ducts (not shown). The connector tube 63 is arranged on the side of a ventilation group and the connector tube 65 is arranged towards the patient. Moreover, the connector tube 65 is preferably provided with a (capnometric) plug 68 connected to the inside of the inner wall 67 and is used to detect the CO₂ content of the flowing gas. Usually, plug 68 is provided with a closing cap (not shown). When the closing cap is not used to close the plug 68, it is inserted in a seat 69 provided on the external surface of the lid 64 in order to avoid the loss of the closing cap. The lids 62, 64 are configured to be coupled together in order to enclose the filter 10 according to Fig. 1 to 5. Detail V of Fig. 7 is shown in Fig. 5.

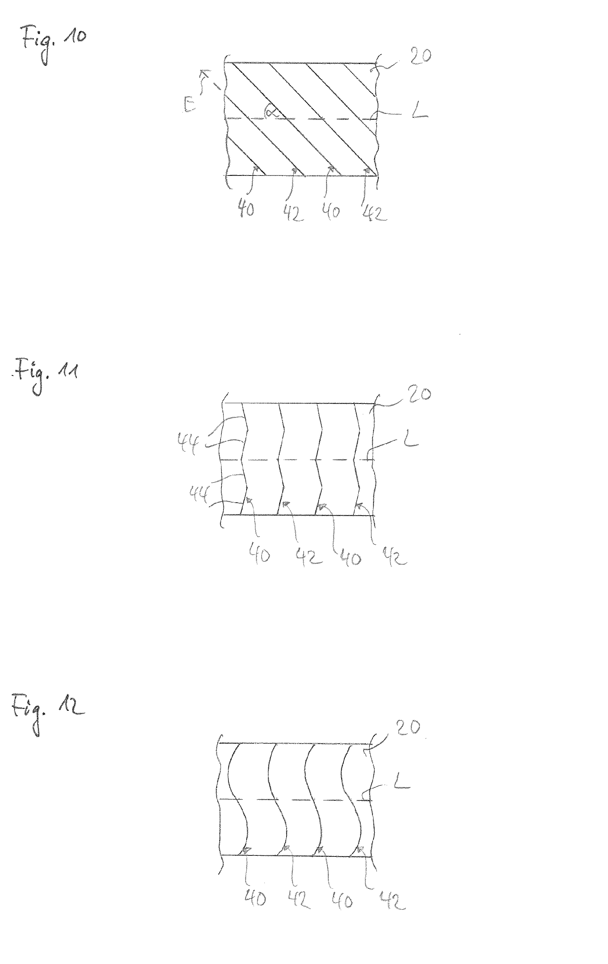

Fig. 10 to 12 are partial plan views similar to Fig. 2 showing alternative directions of extension for the elevations 40, 42. In Fig. 10 the elevations 40, 42 extend in form of parallel lines in an extension direction E diagonally across the sheet 20. The extension direction E is inclined by an angle α relative to the longitudinal direction L of the sheet 20. In Fig. 11 the elevations 40, 42 extend in form of lines, each line having four sections 44. In Fig. 12 the elevations 40, 42 extend in form of a curve across the sheet 20.

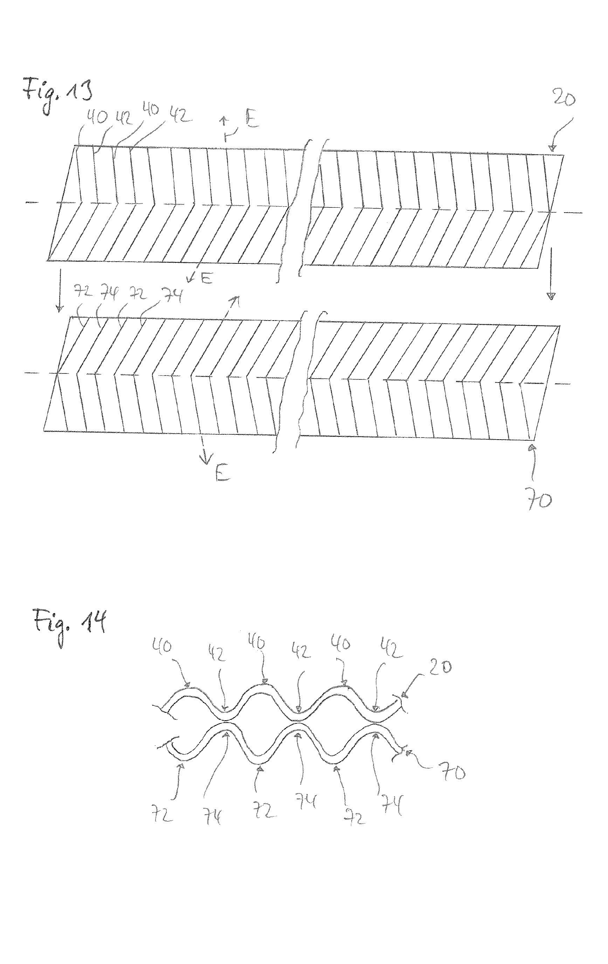

Fig. 13 shows a perspective view of two sheets 20, 70 of filter material to be arranged one upon the other as indicated by the vertical arrows shown in Fig. 13. The upper sheet 20 corresponds to sheet 20 according to Fig. 1. The lower sheet 70 has a plurality of elevations 72, 74 and basically corresponds to the sheet 20. Sheet 70 distinguishes only in the extension direction E of the elevations 72, 74. Both sheets 20, 70 do not comprise any pleats.

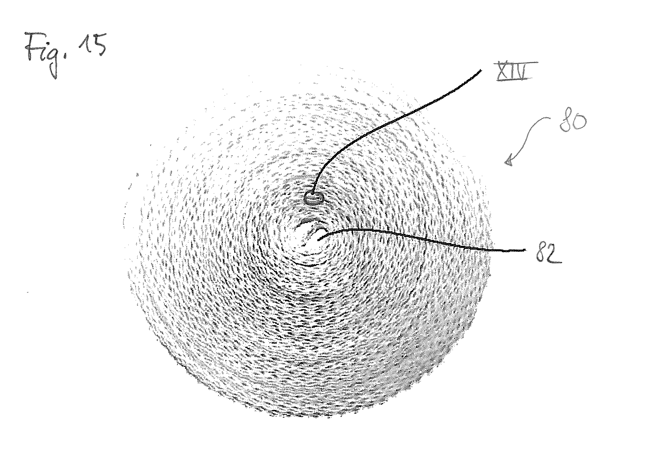

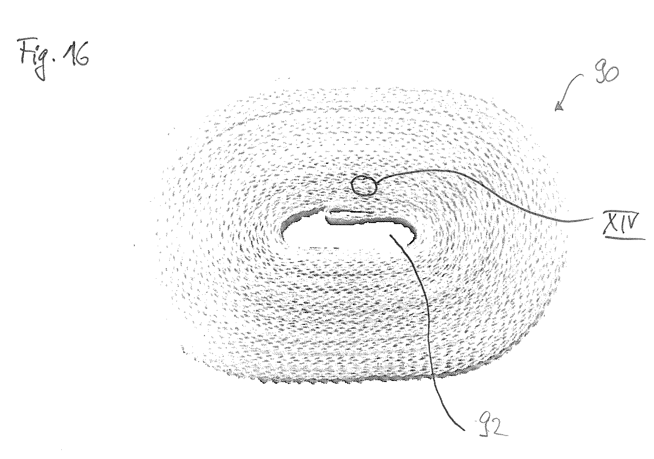

The configuration shown in Fig. 13 is the basis to create the filter devices as shown in Fig. 15 and 16. After pressing a pattern on the two sheets 20, 70 to form the elevations 40, 42, 72, 74 as already explained above, the two sheets 20, 70 are laid one upon the other (cf. arrows in Fig. 13) to build a sandwich assembly such that the elevations 40, 42 one the one hand and elevations 72, 74 on the other hand are directed in different directions relative to each other. Basically, it is possible to connect the two sheets 20, 70 to each other, for example at prescribed locations and using glue, but such a connection is not necessary.

Fig. 14 shows an enlarged partial cross-sectional view of the two sheets 20, 70 being arranged one upon the other. As shown, the elevations 42 of the upper sheet 20 contact the elevations 74 of the lower sheet 70. However, it is sufficient that at least some of all elevations 40, 42, 72, 74 contact each other and it is not necessary that all elevations 40, 42, 72, 74 contact each other. In other words, the elevations 40, 42, 72, 74 function as spacers such that the surface regions of the sheets 20, 70 between the elevations 42 and between the elevations 74 are spaced apart from each other to define a flow passageway for the gas to be filtered. In this way, the gas to be filtered can flow through to gas passageway defined between the two adjacent sheets 20, 70.

To obtain a filter 80 as shown in Fig. 15, the sandwich assembly with the two sheets 20, 70 arranged one upon the other is rolled up. For example the sandwich assembly is rolled up on a cylinder. After rolling up the cylinder can be pulled out and leaves a round hole 82. Thus, the filter 80 has a substantially round shape and can be introduced in a casing having a round or rectangular shape. Detail XIV of Fig. 15 is shown in Fig. 14.

Finally, it is possible to obtain an oval-shaped filter 90 as shown in Fig. 16 by rolling up the sandwich assembly of the two sheets 20, 70 on an oval-shaped cylinder. After rolling up the cylinder can be pulled out and leaves an oval hole 92. Alternatively, the round filter 80 as shown in Fig. 15 can be pressed on the upper and lower sides to reach the oval shape as shown in Fig. 16. Detail XIV of Fig. 16 is shown in Fig. 14. Due to the compressed shape of the filter 90 as shown in Fig. 16 less gas can pass through the filter 90 in the areas below and above the oval through hole 92. In the less compressed areas at the left and right side next to the oval through hole 92 more gas can pass through the filter 90. During use in a clinical environment the oval through hole 92 is filled or blocked to prevent air passing through it.

### List of Reference Signs

- 10: filter

- 20: sheet
- 21: first side
- 22: second side
- 23: first side edge
- 24: second side edge
- 25: first end
- 26: second end
- 28: sheet section

- 30: pleat

- 40: elevation
- 42: elevation
- 44: section

- 50: casing
- 52: first lid
- 54: second lid

- 60: casing
- 62: first lid
- 63: connector tube
- 64: second lid
- 65: connector tube
- 66: outer wall
- 67: inner wall
- 68: plug
- 69: seat

- 70: sheet
- 72: elevation
- 74: elevation

- 80: filter
- 82: hole

- 90: filter
- 92: hole

- E: extension direction (of elevation)
- L: longitudinal direction
- P: extension plane
- T: transverse direction

- α: angle (elevation)
- β: angle (pleat)

- d: distance
- h: height
- l: length
- w: width

## Claims

1. A filter (10, 80, 90) capable of absorbing, retaining and releasing heat and/or moisture of exhaled and inhaled gas, preferably for use in a medical heat and moisture exchanger (HME), the filter (10, 80, 90) comprising at least one sheet (20, 70) of filter material, **characterised in that** the at least one sheet (20, 70) comprises a plurality of elevations (40, 42; 72, 74).

2. The filter (10, 80, 90) according to claim 1, **characterised in that** the at least one sheet (20, 70) comprises a first side (21) and a second side (22), wherein the elevations (40, 42; 72, 74) extend away from the first side (21) and/or second side (22).

3. The filter (10, 80, 90) according to claim 1 or 2, **characterised in that** the elevations (40, 42; 72, 74) are formed integrally with the sheet (20, 70), preferably are made of the same material as the sheet (20, 70).

4. The filter (10, 80, 90) according to one of the preceding claims, **characterised in that** the elevations (40, 42; 72, 74) extend in form of at least one line or curve in an extension direction on the sheet (20, 70).

5. The filter (10, 80, 90) according to claim 4, **characterised in that** the at least one line or curve comprises at least one section (44) extending in an angle (a) relative to a longitudinal direction (L) of the sheet (20, 70), preferably comprises two sections (44) with different directions of extension.

6. The filter (10, 80, 90) according to one of the preceding claims, **characterised in that** the elevations (40, 42; 72, 74) are formed by corrugations.

7. The filter (10, 80, 90) according to one of the preceding claims, **characterised by** at least two sheets (20, 70) of filter material arranged one upon the other.

8. The filter (10, 80, 90) according to claim 7, **characterised in that** the elevations of the adjacent sheets (20, 70) are directed in different directions relative to each other.

9. The filter (10, 80, 90) according to one of the preceding claims, **characterised in that** the at least one sheet (20) has a plurality of pleats (30).

10. The filter (10, 80, 90) according to claim 9, **characterised in that** the at least one sheet (20) has a longitudinal direction (L), wherein the pleats (30) extend substantially transverse to the longitudinal direction (L).

11. The filter (10, 80, 90) according to claim 9 or 10, **characterised in that** the pleats (30) have acute angles (β) and/or are substantially Z-, V- or U-shaped.

12. A method of forming a filter (10, 80, 90) according to any one of the claims 1 to 11 **characterised by** pressing a pattern on the at least one sheet (20, 70) to form the elevations (40, 42; 72, 74).

13. The method according to claim 12, **characterised by** embossing the pattern on the sheet (20, 70), preferably by passing the sheet (20, 70) between two rollers.

14. The method according to claim 12 or 13, **characterised by** folding the sheet (20, 70) against a blade or by rolling up the at least one sheet (20, 70).

15. The method according to claim 12 or 13, **characterised by** lying at least two sheets (20, 70) one upon the other to build a sandwich assembly such that the elevations (40, 42; 72, 74) of the adjacent sheets (20, 70) are directed in different directions relative to each other and rolling up of the sandwich assembly.
